(19) Europäisches Patentamt
European Patent Office
Office européen des brevets

(11) **EP 0 549 915 B1**

(12) **EUROPÄISCHE PATENTSCHRIFT**

(45) Veröffentlichungstag und Bekanntmachung des Hinweises auf die Patenterteilung:
**23.04.1997  Patentblatt 1997/17**

(51) Int Cl.6: **C07K 14/00, A61K 38/00**

(21) Anmeldenummer: **92120727.0**

(22) Anmeldetag: **04.12.1992**

(54) **Neue synthetische Isohirudine mit verbesserter Stabilität**

New synthetic isohirudines with enhanced stability

Nouveaux isohirudines avec stabilité améliorée

(84) Benannte Vertragsstaaten:
**AT BE CH DE DK ES FR GB GR IE IT LI LU NL PT SE**

(30) Priorität: **07.12.1991  DE 4140381**

(43) Veröffentlichungstag der Anmeldung:
**07.07.1993  Patentblatt 1993/27**

(73) Patentinhaber: HOECHST AKTIENGESELLSCHAFT
**65926 Frankfurt am Main (DE)**

(72) Erfinder:
• **Crause, Peter, Dr.**
  **W-6050 Offenbach (DE)**
• **Habermann, Paul, Dr.**
  **W-6239 Eppstein/Ts. (DE)**
• **Tripier, Dominique, Dr.**
  **W-6239 Eppstein/Ts. (DE)**
• **Ulmer, Wolfgang, Dr.**
  **W-6239 Eppstein/Ts. (DE)**
• **Schmid, Gerhard, Dr.**
  **W-8000 München 60 (DE)**

(56) Entgegenhaltungen:
**EP-A- 0 158 986          EP-A- 0 364 942**
**EP-B- 0 207 956**

**Beschreibung**

Die Erfindung betrifft neue synthetische Isohirudine, die durch Austausch im Bereich der Asp-Gly-Motive eine verbesserte Stabilität besitzen. Dadurch wird einerseits die Ausbeute während der Aufarbeitung gesteigert und andererseits eine galenische Zubereitung als direkt injizierbare Fertiglösung ermöglicht.

Aus dem Blutegel Hirudo medicinalis sind medizinisch wertvolle hochaffine Thrombininhibitoren bekannt, von deren Einsatz in der Humanmedizin wesentliche Fortschritte bei der Thrombosetherapie erwartet werden. Diese peptidartigen Inhibitoren werden als Hirudine bezeichnet, wobei eine große Zahl an natürlichen Isohirudinen bekannt ist, die sich nur in wenigen Aminosäuren der Sequenz unterscheiden. Natürliche Isohirudine sind z.B. beschrieben in EP-A 142 860, EP-A 158 564, EP-A 158 986, EP-A 168 342, EP-A 171 024, EP-A 193 175, EP-A 200 655, EP-A 209 061, EP-A 227 938. Die Entwicklung der rekombinanten DNA-Technologie im letzten Jahrzehnt hat es nun ermöglicht, Hirudine im industriellen Maßstab durch den Einsatz gentechnisch modifizierter Mikroorganismen verfügbar zu machen. Verfahren zur Darstellung von Isohirudinen auf der Basis natürlicher Sequenzen sind zum Beispiel beschrieben in EP-A 171 024 und EP-A 200 655 und der darin zitierten Literatur.

Über die therapeutische Wirksamkeit hinaus werden jedoch an ein Pharmakon heutzutage weitergehende Ansprüche gestellt. Dazu gehören u.A. Wirtschaftlichkeit in der Herstellung, klinische Handhabbarkeit und hohe Haltbarkeit im Hinblick auf eine lange Verwendungsdauer.

Damit eine verbesserte Therapie auch unter wirtschaftlichen Gesichtspunkten einer hohen Zahl an Patienten zugute kommen kann, ist es nötig, die Herstellungskosten des Pharmakons klein zu halten. Dies kann bei gentechnischen Produkten durch die Entwicklung optimierter Expressionssysteme, aber auch durch die Anpassung des Pharmakons an ein solches Systems angestrebt werden. In dieser Hinsicht strukturell optimierte Isohirudine sind z.B. beschrieben in EP-A 324 712 und EP-A 448 093.

Dem Aspekt der Verwendungsdauer sollte durch eine hohe Stabilität des Pharmakons Rechnung getragen werden, so daß therapeutischen und pharmakologischen Komplikationen aufgrund der Bildung von Abbauprodukten vorgebeugt wird. Die aus dem Blutegel bekannten Isohirudine als auch Desulfatohirudine aus gentechnisch modifizierten Mikroorganismen lassen in dieser Hinsicht zu wünschen übrig, da sie aufgrund ihrer Struktur zur Bildung von Nebenprodukten durch interne chemische Umwandlung neigen. Auch unter dem Aspekt Wirtschaftlichkeit des Aufarbeitungsverfahrens ist die a priori Vermeidung der Bildung von Nebenprodukten vorteilhaft, da deren Abtrennung entfällt. Dies schlägt sich in verbesserten Ausbeuten nieder.

Eine verbesserte Stabilität würde zudem eine Zubereitung ermöglichen, die eine Lagerung und Anwendung des Pharmakons mit minimalem Aufwand zuläßt. Beim Hirudin stellt eine direkt injizierbare stabile Fertiglösung eine solche Zubereitung dar, die sich dazu noch durch eine lange Verwendbarkeit auszeichnen sollte. Die chemische Instabilität der bekannten Isohirudine ist dabei nämlich ein begrenzender Faktor, da die temperaturabhängige Nebenproduktbildung der gelösten Substanz auch bei Kühlschranklagerung nur eine begrenzte (kurze) Verwendungsdauer einer solchen Zubereitung erlaubt, wobei eine Wirtschaftlichkeit nur schwer erreichbar ist.

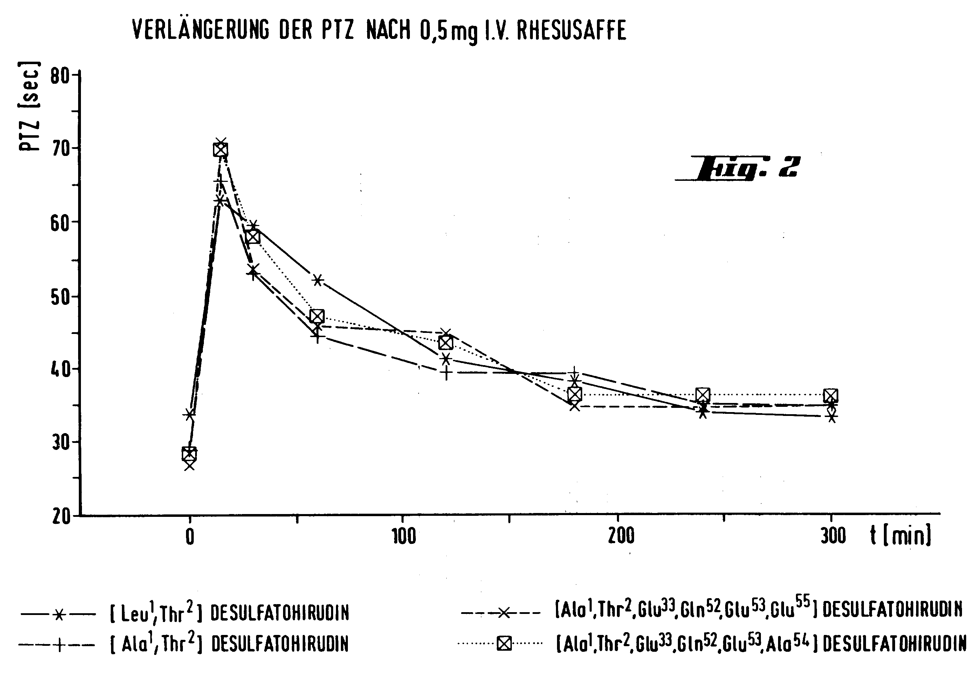

Die bekannten natürlichen Isohirudine und daraus abgeleiteten Desulfatohirudine für die pharmazeutische Entwicklung unterscheiden sich nur in wenigen Aminosäurebausteinen, wobei in der Sequenz variable und konservative Regionen unterschieden werden können. Zu den konservativen Regionen gehören die Sequenzen -Ser$^{32}$-Asp(Asn)$^{33}$-Gly$^{34}$- und -Asn$^{52}$-Asp(Asn)$^{53}$-Gly$^{54}$-Asp$^{55}$-. Die Numerierung erfolgt nach der durch Dodt et al., FEBS LETTERS 165 (1984) 180 - 183 publizierten Sequenz mit 65 Aminosäuren. Proteinchemische Analysen von Hirudin-Abbauprodukten haben nun gezeigt, daß diese Sequenzmotive zum überwiegenden Teil für die chemische Instabilität von Hirudin verantwortlich sind. Die Desamidierung von Asparagin zu Asparaginsäure kann dabei außer Betracht gelassen werden, da sie zu einer weiteren natürlichen Isohirudinstruktur führt. Wesentlich für den Hirudinabbau ist die Isomerisierung und Racemisierung an den beiden -Asp-Gly-Sequenzen. Die Bedeutung dieser Reaktionen für den Abbau von Proteinen ist literaturbekannt (JBC 262, 1987, S. 785-793 u. JBC 264, 1989, S. 6164-6170). Die Bedeutung der carboxyterminalen Struktur des Hirudins für die hochaffine Bindung an Thrombin ist ebenfalls bekannt. Ein Aminosäureaustausch in Bereichen mit konservativem Sequenzcharakter, die an der Bindung beteiligt sind, ist mit der Gefahr einer Affinitätseinbuße verbunden. Überraschenderweise wurde nun gefunden, daß bestimmte stabilitätsverbessernde Modifikationen in konservativen Bereichen vorgenommen werden können, ohne die Affinität zum Thrombin und damit die Wirksamkeit zu beeinträchtigen (Beispiel 7, Tab. 1 und Beispiel 8, Fig. 2).

Zudem zeigt sich überraschenderweise, daß trotz der vorgenommenen Austausche, auch bei mehr als einer Aminosäure keine Zunahme der Antigenität eintritt.

Ferner wurde gefunden, daß beide -Asp-Gly-Sequenzen gleichermaßen zur Instabilität des Hirudins beitragen. Erst durch Modifikation beider Sequenzbereiche kann die Nebenproduktbildung entscheidend reduziert werden (Beispiel 9, Tabelle 2).

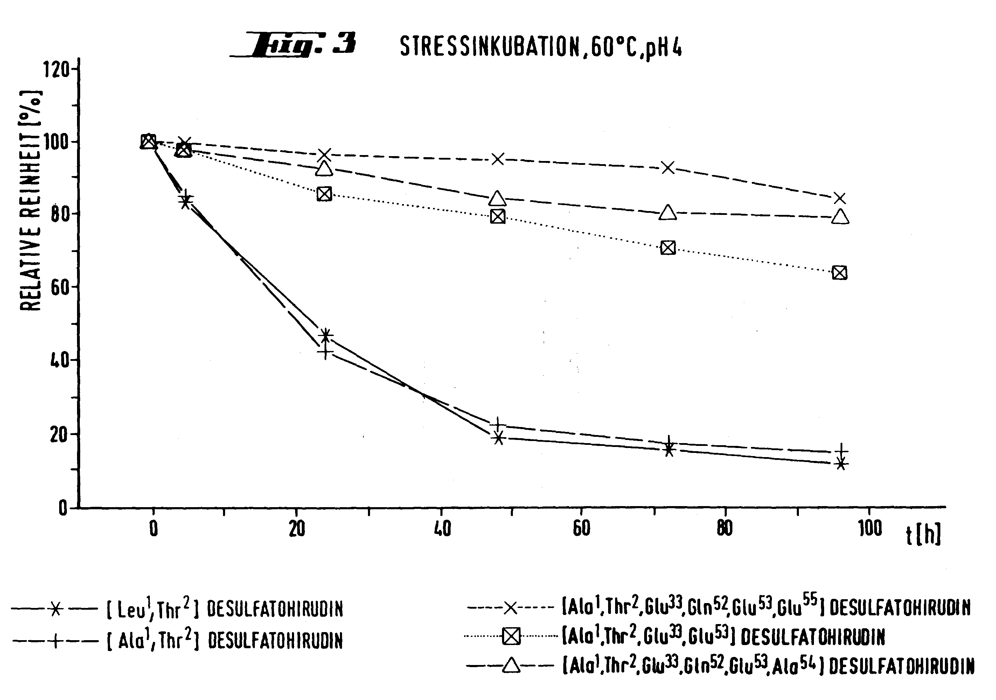

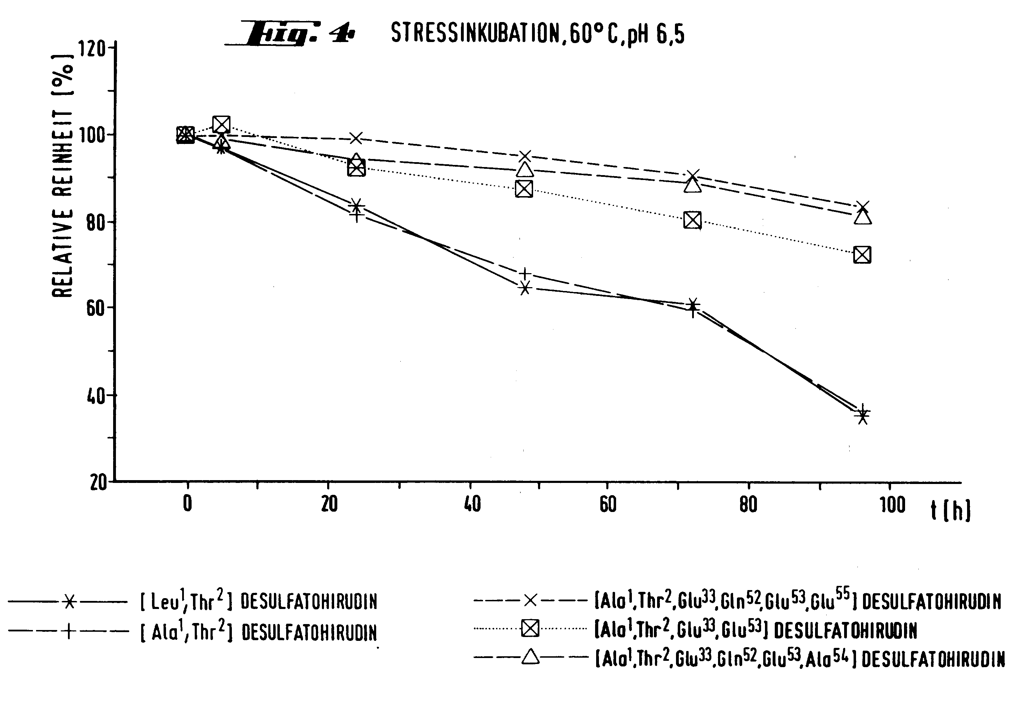

Der zeitliche Verlauf der Nebenproduktbildung der Stammverbindungen als auch der optimierten Desulfatohirudine sowohl bei saurem (Beispiel 11, Fig.3) als auch bei physiologischem pH (Beispiel 10, Fig.4) zeigt, daß der Aminoterminus keinen Einfluß auf die Stabilität hat und eine Modifikation somit nicht zur Stabilisierung beiträgt. Daraus folgt,

2

daß die am Beispiel des [Ala[1],Thr[2]] Desulfatohirudins gezeigten Optimierungen auf Isohirudine mit unterschiedlichem Aminoterminus übertragbar sind (z.B. [Val[1],Val[2]]- und [Ile[1],Thr[2]]- Desulfatohirudin).

Eine detaillierte Analytik der Stabilität verschiedener synthetischer Isohirudine (Fig.3 und 4) offenbart jedoch, am Beispiel von [Ala[1],Thr[2],Glu[33],Glu[53]] Desulfatohirudin, daß ein einfacher Austausch der Asparaginsäure nicht den optimalen Effekt liefert. Die überlegene Stabilität von [Ala[1],Thr[2],Glu[33],Gln[52],Glu[53],Ala[54]]-Desulfatohirudin und [Ala[1],Thr[2], Glu[33],Gln[52],Glu[53],Glu[55]]-Desulfatohirudin -Desulfatohirudin zeigt überraschenderweise, daß der zusätzliche Austausch von Asparagin gegen Glutamin an Position 52 einen wesentlichen Beitrag zur Stabilisierung leistet.

Die Erfindung betrifft folglich Isohirudine mit verbesserter Stabilität, wobei an Position 33 Glu, an Position 52 Gln, Glu, Asn oder Asp, an Position 53 Glu und an Position 54 Gly oder Ala und an Position 55 Glu oder Asp steht.

Bevorzugt sind Verbindungen der Formel I

$$
\begin{array}{l}
\phantom{xxxxxxxxxxxxxxxxxxxxxxxxxxx}10\\[4pt]
A^1\phantom{xxx}-A^2\text{-Tyr-Thr-Asp-Cys-Thr-Glu-Ser-Gly-Gln-Asn-Leu-Cys-Leu-}\\[4pt]
\phantom{xxxxxxx}20\phantom{xxxxxxxxxxxxxxxxxxxxxxxxxxxxxxxxxx}30\\[4pt]
\text{Cys-Glu-Gly-Ser-Asn-Val-Cys-Gly-Gln-Gly-Asn-Lys-Cys-Ile-Leu-}\\[4pt]
\phantom{xxxxxxxxxxxxxxxxxxxxxxxxxxxx}40\\[4pt]
\text{Gly-Ser- B - Gly-Glu-Lys-Asn-Gln-Cys-Val-Thr-Gly-Glu-Gly-Thr-}\\[4pt]
\phantom{xxxxxxxx}50\phantom{xxxx}52\phantom{xx}53\phantom{xx}54\phantom{xx}55\phantom{xxxxxxxxx}60\\[4pt]
\text{Pro-Lys-Pro-Gln-Ser-His- C - D - E - F -Phe-Glu-Glu-Ile-Pro-}\\[4pt]
\text{Glu-Glu-Tyr-Leu-Gln}
\end{array}
$$

wobei

A[1]     = Leu, Ala, Ile oder Val bedeutet
A[2]     = Thr oder Val
B       Glu bedeutet
C       Gln oder Glu bedeutet
D       Glu bedeutet
E       Gly oder Ala bedeutet
und F    Asp oder Glu bedeutet.

Besonders bevorzugt sind die Verbindungen [Ala[1] oder Leu[1], Thr[2], Glu[33], Gln[52]-Glu[53]-Ala[54]-] Desulfatohirudin und [Ala[1] oder Leu[1], -Thr[2], Glu[33], Gln[52]-Glu[53], Glu[55]-] Desulfatohirudin.

Die erfindungsgemäßen synthetischen Hirudinderivate können über Mikroorganismen oder aber auch chemisch synthetisch hergestellt werden. Bevorzugt ist die Herstellung in Bäckerhefe oder E.coli.

Der chemische Stabilität der optimierten Isohirudine in Verbindung mit einem hocheffizienten Expressionssystem erlaubt relativ einfache und damit kostengünstige Aufarbeitungsverfahren. Dabei kann die Kombination an sich bekannter biochemischer Methoden zu Verfahren führen, die sich leicht voneinander unterscheiden können. Solche Verfahrensvarianten sind ebenfalls Gegenstand der Erfindung. Abkömmlinge des [Ala[1],Thr[2]] Desulfatohirudins können beispielsweise in einem Expressionssystem gemäß der Europäischen Patententanmeldung EP-A 448 093 hergestellt werden, wobei die Aufarbeitung aus nur wenigen Schritten besteht: Die Zellsuspension am Ende der Fermentation oder ein zellfreies Kulturfiltrat wird auf pH 2-4 (z.B. mit Ameisensäure, HCl) angesäuert, ein dabei anfallendes proteinhaltiges Präzipitat kann abzentrifugiert oder abfiltriert werden. Aus dem klaren Überstand kann das Produkt im einfachsten Fall, sofern dies das verwendete Kulturmedium zuläßt, über Reversed Phase -Chromatographie hoch angereichert werden. Bei Verwendung komplexer Medien hingegen ist es vorteilhaft, den Salzgehalt der Lösung zu reduzieren, z.B. durch Ultra-/Diafiltration, um dann eine Ionenaustauschchromatographie an einem Kationenaustauscher, z.B. Fractogel® EMD- SO$_3^-$ vorzunehmen. Anstelle der Ultrafiltration kann auch eine hydrophobe Adsorption an ein geeignetes Harz, wie z.B. beschrieben in der Europäischen Patentanmeldung EP-A 316 650 durchgeführt werden. Das Produkt der Kationenaustauschchromatographie kann dann direkt in die Reversed Phase -Chromatographie (z. B. an Lichroprep® RP18) eingeschleust werden, die ein hochreines Produkt liefert. Im Bedarfsfall können verbliebene Verunreinigungen durch eine zusätzliche Anionenaustauschchromatographie, z.B. an Q-Sepharose, entfernt werden. Durch entsprechende Optimierungen der einzelnen Stufen können Ausbeuten > = 50% realisiert werden.

Bei Verwendung eines Expressionssystems gemäß der Europäischen Patentanmeldung EP-A 316 650 zur Darstellung von stabilisierten Abkömmlingen des [Leu¹,Thr²]-Desulfatohirudins kann die Aufarbeitung analog durchgeführt werden, wobei es sich jedoch empfiehlt, das Produkt vor der Kationenaustauschchromatographie durch einen Batch-Anionenaustauscherschritt, wie z.B. beschrieben in der Europäischen Patentanmeldung EP-A 316 650, weiter anzureichern.

Beispiel 1: Konstruktion des Vektors pCMT203

Biotechnologische Arbeiten wie das Anlegen von Saatzellbänken oder die Fermentation eines Stammes werden bevorzugt unter Selektionsdruck auf das bearbeitete Wirts-/Vektorsystem durchgeführt. Damit wird die Gefahr der Verunreinigung durch fremde Mikroorganismen minimiert. Nach den Richtlinien der amerikanischen Gesundheitsbehörde sollte das Antibiotikum Ampicillin nicht in Herstellungsverfahren für rekombinante Proteine Verwendung finden.

In der Europäischen Patentanmeldung EP-A 448 093 wurden die Plasmide pCM7051 und pCM7053 beschrieben. Eine Verbesserung der Plasmide im Hinblick auf den oben beschriebenen Aspekt kann dann erreicht werden, wenn man die Vektor-DNA um die bekannte Resistenz gegen Tetrazyklin erweitert.

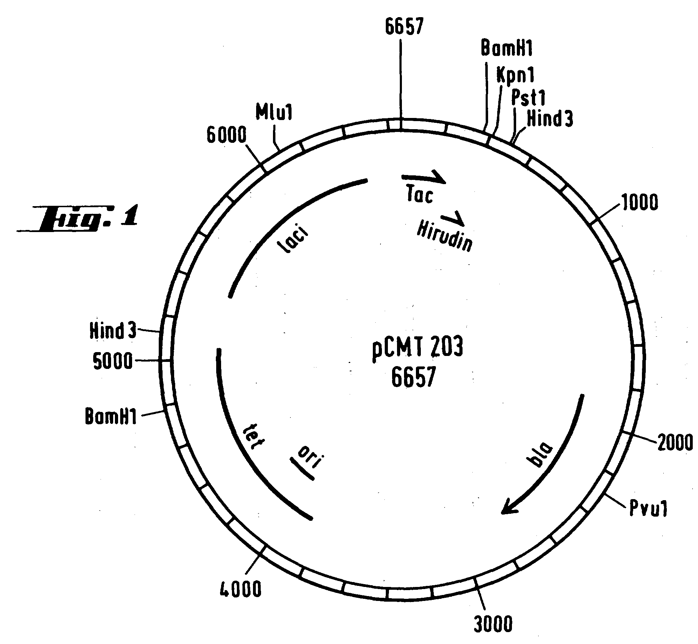

Dazu wird DNA des Plasmides pCM7051 mit Nru1 linearisiert und dem isolierten 1,1kb- Nru1-Fragment aus dem Plasmid pCM7053 ligiert. Dieses DNA-Fragment enthält den im Plasmid pCM7051 fehlenden 5'-terminalen Teil des Tetrazyklinresistenzgenes. Kompetente Zellen des E.coli-Stammes Mc1061 werden mit dem Ligationsgemisch transformiert und auf NA-Agarplatten, die 12,5 mg/l Tetrazyklin enthalten, ausplattiert. Nach Inkubation bei 37oC über Nacht erhält man Transformanten. Plasmid-DNA wird davon reisoliert und mittels Restriktionsanalyse charakterisiert. Die DNA eines richtigen Plasmides wird anschließend mit den Enzymen Ava1 und Nde1 nach Angaben der Hersteller umgesetzt und gelelektrophoretisch aufgetrennt. Das größere der beiden Fragmente wird isoliert und die überhängenden Enden mit Hilfe von Klenow Polymerase aufgefüllt. Anschließend wird das Fragment autoligiert und erneut nach E.coli Mc1061 transformiert. Nach Charakterisierung der DNA mittels Restriktionsanalyse erhalten die gewünschten Plasmide die Bezeichnung pCMT203 (Fig.1).

Beispiel 2: Konstruktion von Hirudin-Varianten mit Alanin als N-terminaler Aminosäure

Hirudin-Varianten,die mit Alanin beginnen,sollen in E.coli exprimiert werden. Zur Klonierung wurden die in Beipiel 1 beschriebenen Vektoren pCM7053, pCMT203 und das in EP-A 171 024 beschriebene Plasmid der Figur 3 mit der als DNA-Sequenz I bezeichneten synthetischen DNA-Sequenz für Hirudin verwendet. Dieses Plasmid wird im folgenden als Plasmid pK152 bezeichnet. Ferner wurden unter Einsatz der DNA-Synthese-Maschine "391 DNA Synthesizer" der Firma Applied Biosystems folgende Oligonukleotide synthetisiert:

Hir1    5'- CCCGAAACCGCAGTCTCACCAGGAAGGCGAATT - 3'

Hir2    5'- CGAATTCGCCTTCCTGGTGAGACTGCGGTTTCGGGGGTAC - 3'

Hir5    5'- GATCCGAAGGTGAAAAGAACCAGTGCGTTACTGGCGAAGGTAC-3'

Hir6    5'- CTTCGCCAGTAACGCACTGGTTCTTTTCACCTTCG - 3'

Hir13   5'- CCCGAAACCGCAGTCTCATAACGAGGGCGACTT - 3'

Hir14   5'- CGAAGTCGCCCTCGTTATGAGACTGCGGTTTCGGGGGTAC - 3'

Hir15   5'- CCCGAAACCGCAGTCTCATCAGGAGGCTGACTT - 3'

Hir16  5'- CGAAGTCAGCCTCCTGATGAGACTGCGGTTTCGGGGTAC - 3'

Bespiel 2a: Konstruktion der Hirudin-Variante 13 (Ala$^1$,Glu$^{33}$,Gln$^{52}$,Glu$^{53}$,Glu$^{55}$) in Plasmid pSCH13

DNA des Plasmides pK152 wird mit den Enzymen BamH1 und Kpn1 umgesetzt und die beiden enstehenden Fragmente gelelektrophoretisch voneinander getrennt. Das große Fragment wird isoliert und in einer T4-DNA-Ligasereaktion mit den zuvor zum Doppelstrang hybridisierten Oligonukleotidsequenzen Hir5 und Hir6 umgesetzt. Kompetente E.coli Mc1061 Zellen werden mit dem Ligationsgemisch transformiert. Parallel dazu wird das pK153 Vektorfragment in einer Autoligationsreaktion mit sich selbst ligiert und ebenfalls transformiert. Die Transformationsansätze werden auf NA-Platten, die 20mg/l Ampicillin enthalten, ausplattiert und über Nacht bei 37°C inkubiert. Am nächsten Morgen wird das Experiment ausgewertet. Die Klonierung wird dann als erfolgversprechend betrachtet, wenn die Ligation mit dem Oligonukleotidfragment mindestens 100-fach mehr Transformanten liefert als die Autoligation. Von Transformanten der Klonierungsreaktion wird dann Plasmid-DNA isoliert und mittels Restriktionsanalyse charakterisiert. Von Plasmid-DNA, die das richtige Restriktionsmuster zeigt, wird das BamH1/Hind3 Fragment, das die Hirudinpeptidsequenz 32-65 mit dem Austausch Asp$^{55}$ nach Glu$^{55}$ enthält, isoliert. Dieses Fragment wird mit dem BamH1/Hind3 geöffneten Vektor pCM7053 ligiert. Es entsteht das Plasmid pCM7053Var3. Das Plasmid enthält die DNA-Sequenz für ein verändertes Hirudin, das in Position 55 die Aminosäure Glu trägt.

DNA des Plasmides pK152 wird mit den Restriktionsenzymen Kpn1 und Bstb1 gespalten. Nach gelelektrophoretischer Auftrennung wird das große Vektorfragment isoliert. Dieses Fragment wird mit den zuvor zu Doppelstrang hybridisierten Oligcnukleotiden Hir1 und Hir2 ligiert. Entsprechend dem oben beschriebenen Ablauf entsteht ein Derivat des Plasmides pK153. Dieses wird als Variante 1 bezeichnet. Von diesem Plasmid wird das BamH1/Hind3 Fragment isoliert, das mit dem BamH1/Hind3 geöffneten Vektor pCM7053 ligiert wird. Es entsteht das Plasmid pCM7053Var1, welches für ein verändertes Hirudin codiert, das in Position 52,53 und 55 die Aminosäuren Gln, Glu und Glu enthält. Das Plasmid zeichnet sich durch eine im Vergleich zu pCM7053 zusätzlich vorhandene Erkennungsstelle für das Restriktionsenzym EcoR1 aus.

DNA der Plasmide pCM7053Var1 und pCM7053Var3 wird mit den Enzymen Kpn1 und Mlu1 doppelverdaut und gelelektrophoretisch aufgetrennt. Es entstehen jeweils zwei Fragmente von denen im Falle des pCM7053Var1 Ansatzes das größere der beiden und im Falle von pCM7053Var3 das kleinere der beiden Fragmente isoliert wird. Beide Fragmente werden in einer Ligation zu dem neuen Plasmid pVar13 vereinigt, das in dem Stamm E.coli Mc1061 exprimiert wird. Hirudin wird gemäß Beispiel 5 isoliert und mittels Aminosäureanalyse charakterisiert. Mit der erwarteten Aminosäurezusammensetzung ist die Richtigkeit der Konstruktion des Plasmides pVar13 bestätigt.

DNA der Plasmide pCMT203 und pVar13 wird nun mit den Restriktionsenzymen Mlu1 und Pvu1 umgesetzt und gelelektrophoretisch aufgetrennt. Es entstehen jeweils zwei Fragmente von denen jeweils das größere isoliert wird. Die so isolierten Fragmente werden in einer Ligasereaktion zu dem Plasmid pSCH13 vereinigt. Durch Restriktionsenzymanalyse und DNA-Sequenzanalyse wird dessen Struktur bestätigt. Dieses Plasmid wird in die in der Europäischen Patentanmeldung EP-A 448 093 beschriebenen E.coli K12 Sekretormutanten durch Transformation in bekannter Weise eingeführt.

Beispiel 2b: Konstruktion der Hirudin-Variante 83 (Ala$^1$,Glu$^{33}$,Glu$^{53}$) in Plasmid pSCH83

Das in Beispiel 2a beschriebene Kpn1/Bstb1 - pK152-Vektorfragment wird mit den zuvor zu Doppelstrang hybridisierten Oligonukleotiden Hir13 und Hir14 ligiert. Es entsteht ein als Variante 8 bezeichnetes Plasmid. Von diesem Plasmid wird gemäß Beispiel 2a das BamH1/Hind3 -Fragment isoliert und in den BamH1/Hind3 geöffneten Vektor pCM7053 eingeführt. Es entsteht das Plasmid pCMVar8, von dem das kleinere Kpn1/Mlu1 -Fragment isoliert wird. Dieses wird mit dem großen Kpn1/Mlu1 - Fragment aus Plasmid pCMVar3 ligiert. Es entsteht das Plasmid pVar83, das in dem Stamm E.coli Mc1061 exprimiert wird. Nach Isolation des Hirudinderivats und anschließender Aminosäureanalyse bestätigt sich die Plasmidstruktur, so daß gemäß Beispiel 2a das Mlu1/Pvu1-Fragment isoliert wird, das mit dem dem großen Mlu1/Pvu1-Vektor -Fragment aus Plasmid pCMT203 zu Plasmid pSCH83 ligiert wird. Dieses Plasmid wird in die oben beschriebenen Sekretormutanten eingeführt.

Beispiel 2c: Konstruktion der Hirudin-Variante 93 (Ala$^1$,Glu$^{33}$,Gln$^{52}$,Glu$^{53}$,Ala$^{54}$) in Plasmid pSCH93

Die Konstruktion des Plasmides pSCH93 erfolgt in Analogie zu Beispiel 2b. Dabei wird im ersten Klonierungsschritt das Bstb1/Kpn1 -pK152-Fragment mit den zu Doppelstrang hybridisierten Oligonukleotiden Hir15 und Hir16 zu dem Plasmid mit der Bezeichnung Variante 9 umgesetzt.

Beispiel 3: Expression der Plasmide pSCH13, pSCH83 und pSCH93

Die Expression der Plasmide pSCH13, pSCH83und pSCH93 erfolgt sowohl im Schüttelkolben als auch im 10 Litermaßstab gemäß der Europäischen Patentanmeldung EP-A 448 093. Dabei finden die beschriebenen Stämme oder Varianten davon Anwendung. Für die Expression von Kulturen im Kubikmetermaßstab können sich naturgemäß veränderte Medien, Induktionsbedingungen und Fermentationszeiten ergeben, was dem Fachmann bekannt ist.

Beispiel 4: Klonierung und Expression der Hirudinvarianten 13 und 93 in Bäckerhefe

In der Europäischen Patentanmeldung EP-A 324 712 wird ein synthetisches Hirudin beschrieben, das in Abwandlung der natürlichen Sequenz N-terminal die Aminosäure Leucin trägt. Dieses Hirudin kann ebenfalls weiter optimiert werden, wenn man in der dem Leucin folgenden Sequenz ab Aminosäure 2 die in den zuvor für die Varianten 13 bzw. 93 beschriebenen Veränderungen vornimmt. Dabei wird beispielhaft auf die in dieser Anmeldung beschriebenen Vektoren und Stämme zurückgegriffen. Es ist dem Fachmann geläufig, daß auch jedes andere Hefeexpressionssystem, das zur Ausschleusung von Hirudin oder Varianten hiervon führt, benutzt werden kann.

Der in der Europäischen Patentanmeldung EP-A 324 712 beschriebene Klonierungsvektor 7 wird mit BamH1 und Hind3 geöffnet und jeweils mit dem aus Plasmid pSCH13 bzw. pSCH93 isolierten BamH1/Hind3 Fragment, das die jeweils im Klonierungsvektor fehlenden Aminosäuren des carboxyterminalen Teiles der Hirudinsequenz umfaßt, ligiert. Es entstehen die Plasmide p713 und p793, die mittels Restriktionsanalyse charakterisiert werden. Von richtiger DNA dieser Plasmide wird anschließend das EcoR1/Hind3 -Fragment isoliert und die überstehenden Enden in einer Klenow-Polymerasereaktion aufgefüllt. Die so präparierten Fragmente werden jeweils mit dem stumpfendigen Vektorfragment von Plasmid yEP13 gemäß Beispiel 1 der Europäischen Patentanmeldung EP-A 324 712 ligiert. Es entstehen die Plasmide pHABVar131 und pHABVar132, die sich nur in Bezug auf die Orientierung des insertierten Fragmentes unterscheiden und die für ein Hirudinderivat kodieren, das durch die Aminosäuren $Leu^1$, $Glu^{33}$, $Gln^{52}$, $Glu^{53}$ und $Glu^{55}$ gekennzeichnet ist, sowie die Plasmide pHABVar931 und pHABVar932, die sich ebenfalls nur durch die Orientierung des insertierten Fragmentes unterscheiden und für ein Hirudinderivat kodieren, das durch die Aminosäuren $Leu^1$, $Glu^{33}$, $Gln^{52}$, $Glu^{53}$ und $Ala^{54}$ gekennzeichnet ist. Die Plasmide werden in die in der Anmeldung beschriebenen Hefestämme beispielhaft transformiert. Die Expression und Reinigung der Hirudin-Derivate kann gemäß der dort beschriebenen Prozedur durchgeführt werden. Bekanntermaßen kann bei der Reinigung auf die Zentrifugation und die sich anschließende Adsorptionschromatographie verzichtet werden, wenn z.B. das Pellicon Ultrafiltrationssystem der Firma Millipore verwendet wird. Die hier verwendeten Methoden sind für den Labormaßstab beschrieben. Für Kulturen im Kubikmetermaßstab können andere Fermentationszeiten, Kulturbedingungen sowie Schritte der Aufarbeitung notwendig werden. Dies ist dem Fachmann bekannt.

Beispiel 5: Reinigung von $[Ala^1, Thr^2, Glu^{33}, Gln^{52}, Glu^{53}, Glu^{55}]$-Desulfatohirudin

Ein zellfreier Kulturüberstands mit 3,6 g Hirudin per l wurde durch Zugabe von Ameisensäure auf pH 3 gestellt. Nach 1 h bei RT wurde das enstandene Präzipitat über eine CEPA-Zentrifuge abgeschleudert. Die Leitfähigkeit des klaren Überstand wurde durch Diafiltration auf < = 2,5 mS/cm abgesenkt. Danach wurde das Produkt durch aufeinanderfolgende Chromatographieschritte an Fractogel® EMD-$SO_3$, Lichroprep® RP18 und Q-Sepharose hochrein dargestellt.

Verbliebene Salze und Pufferbestandteile wurden aus dem Q-Sepharose-Eluat durch kombinierte Ultra-/Diafiltration entfernt, wonach das Produkt durch Lyophilisation als Trockensubstanz gewonnen werden konnte.

Beispiel 6: Reinigung von $[Ala^1, Thr^2, Glu^{33}, Gln^{52}, Glu^{53}, Ala^{54}]$-Desulfatohirudin

Am Ende der Fermentation wurde die Kulturlösung in Gegenwart- der Zellsubstanz auf pH 3 angesäuert. Zellmasse und anfallendes Präzipitat wurden über einen Separator entfernt. Zu dem klaren Überstand wurden 5 % w/v Diaion HP20 gegeben, wobei eine quantitative Adsorption des Hirudins resultierte. Nach Entfernung der Mutterlauge durch Filtration wurde das Harz einmal wäßrig gewaschen. Danach wurde das Produkt mit auf pH3 angesäuertem 30 %igem Isopropanol desorbiert. Das klare Eluat wurde wie in Beispiel 5, beginnend mit der Kationenaustauschchromatographie, weiterverarbeitet, um dann nach Lyophilisation ein hochreines Trockenprodukt zu erhalten.

Beispiel 7: Vergleichende $K_1$-Wert-Bestimmung optimierter Isohirudine

$K_1$-Werte wurden nach der Methode von Stone und Hofsteenge (Biochemistry 25, S.4622-4628, 1986) bestimmt: 0.2 ml einer 1.25 mM Lösung von D-HHT-Gly-Arg-pNA wurden mit 1,7 ml 0,1 M Tris, 0,2 M NaCl und 0,1 % (v/v) Triton X-100 pH 8,3 und 0,1 ml des zu testenden Isohirudins in 145 mM NaCl auf 25°C temperiert. Die Bindung wurde durch Zugabe von 0,05 ml Thrombinlösung gestartet. Die Absorption bei 405 nm wurde über eine Periode von 10-20 min aufgezeichnet.

Die Reaktion folgt der Gleichung:

$$[P] = v_s*t + (v_o-v_s)(1-e^{-k*t})/k + d,$$

wobei

[P] = Produktkonzentration (Nitroanilin)
$v_o$ = initiale Geschwindigkeit
$v_s$ = Reaktionsgeschwindigkeit im Gleichgewichtszustand
d    = [P] bei t=0

Die Geschwindigkeitskonstante k wurde durch nichtlineare Regression bestimmt. Die Extrapolation von k bei verschiedenen Inhibitorkonzentrationen auf [I] = 0 entsprechend

$$k = k_{on}*[I]/(1+S/K_m) + k_{off}$$

liefert die Geschwindigkeitskonstanten $k_{on}$ und $k_{off}$ und damit $K_i=k_{off}/k_{on}$.

Tabelle 1

| $K_i$-Werte optimierter Desulfatohirudine | |
| --- | --- |
| Verbindung | $K_{i(app.)}$ [M] |
| [Ile[1],Thr[2]] Desulfatohirudin[1] | $6,1x10^{-10}$ |
| [Leu[1],Thr[2]] Desulfatohirudin[2] | $1,4x10^{-10}$ |
| [Ala[1],Thr[2]] Desulfatohirudin[3] | $2,0x10^{-10}$ |
| [Ala[1],Thr[2],Glu[33]] Desulfatohirudin | $1,6x10^{-10}$ |
| [Ala[1],Thr[2],Gln[52],Glu[53],Glu[55]] Desulfatohirudin | $1,9x10^{-10}$ |
| [Ala[1],Thr[2],Glu[33],Gln[52],Glu[53],Glu[55]] Desulfatohirudin | $3,0x10^{-10}$ |
| [Ala[1],Thr[2],Ala[54]] Desulfatohirudin | $2,7x10^{-10}$ |
| [Ala[1],Thr[2],Glu[33],Ala[54]] Desulfatohirudin | $3,5x10^{-10}$ |
| [Ala[1],Thr[2],Glu[53]] Desulfatohirudin | $3,8x10^{-10}$ |
| [Ala[1],Thr[2],Glu[33],Glu[53]] Desulfatohirudin | $3,7x10^{-10}$ |
| [Ala[1],Thr[2],Gln[52],Glu[53],Ala[54]] Desulfatohirudin | $2,2x10^{-10}$ |
| [Ala[1],Thr[2],Glu[33],Gln[52],Glu[53],Ala[54]] Desulfatohirudin | $3,1x10^{-10}$ |

[1] Von einem natürlichen Isohirudin abgeleitetes Desulfatohirudin.

[2] Optimiertes Desulfatohirudin für Hefeexpression laut EP-A 324 712.

[3] Optimiertes Desulfatohirudin für die E.coli-Sekretionsexpression laut EP-A 448 093.

Beispiel 8: Einfluß optimierter [Ala[1],Thr[2]] Desulfatohirudin-Analoge auf die partielle Thromboplastinzeit (PTZ) im Rhesusaffen

[Leu[1],Thr[2]] Desulfatohirudin, [Ala[1],Thr[2]] Desulfatohirudin, [Ala[1],Thr[2],Glu[33],Gln[52],Glu[53],Glu[55]] Desulfatohirudin und [Ala[1],Thr[2],Glu[33],Gln[52],Glu[53],Ala[54]] Desulfatohirudin wurden in einer Dosis von 0,5 mg/kg männlichen Rhesusaffen mit einem Körpergewicht von 6,5 +- 1,6 kg intravenös appliziert. In bestimmten Abständen wurden dann Blutproben für die Bestimmung von Gerinnungsparametern entnommen. Die partielle Thromboplastinzeit (PTZ) wurde wie folgt bestimmt (Abb. 2):

0,1 ml Citratplasma und 0,1 ml PTT-Reagenz aus Human-Thrombozyten (Behringwerke) wurden in einem auf 37°C vorgewärmten Teströhrchen zusammengegeben und zur vollständigen Aktivierung des endogenen Gerinnungssystems genau für 2 min bei 37°C gehalten. Anschließend wurde 0,1 ml 0.025 M Calciumchloridlösung hinzugegeben und die Gerinnung im Koagulometer (nach Schnitger und Gross) gemessen.

Beispiel 9: 20 Stunden-Stabilität von [Ala[1],Thr[2]] Desulfatohirudin-Analogen bei pH 7 und 60°C

Die zu testende Verbindung wurde in 20 mM NaP$_i$ pH 7, 300 mM NaCl zu 0,5 mg/ml gelöst und für 20 h bei 60°C inkubiert. Zu den Zeitpunkten t=0 und t=20h wurden Proben entnommen und per RP-HPLC (Nucleosil®) sowie Anionenaustauschchromatographie (Mono Q®) analysiert. Der Anteil neugebildeter Nebenprodukte wurde kalkuliert.

Tabelle 2:    Stabilität optimierter Desulfatohirudine unter Stressbedingungen: 20 h, 60°C, pH 7

| Verbindung | % neugebildete Nebenprodukte |
|---|---|
| [Ala[1],Thr[2]] Desulfatohirudin[1] | 23,0 |
| [Ala[1],Thr[2],Glu[33]] Desulfatohirudin | 15,2 |
| [Ala[1],Thr[2],Gln[52],Glu[53],Glu[55] ] Desulfatohirudin | 14,6 |
| [Ala[1],Thr[2],Glu[33],Gln[52],Glu[53],Glu[55]] Desulfatohirudin | 3,2 |

[1]  Stammverbindung

Beispiel 10: Stabilität von [Ala[1],Thr[2]] Desulfatohirudin-Analogen bei pH 6,5 und 60°C

Die gereinigten Isohirudine wurden als Lyophilisat zu 1 mg/ml in Wasser gelöst und mit 1M Na$_2$HPO$_4$ auf pH 6,5 gestellt. Nach Sterilfiltration wurden die Lösungen im Schüttelwasserbad unter Lichtausschluß bei 60°C inkubiert. Proben wurden zu den Zeitpunkten t = 0,5,24,48,72 und 96 h entnommen und per RP-HPLC (Nucleosil®) und Ionenaustauschchromatographie (Mono Q®) auf ihren Gehalt an Nebenprodukten analysiert. Die Reinheit zum Zeitpunkt t = x ist dargestellt relativ zur Reinheit t = 0, wobei aus den beiden Analysesystemen jeweils der ungünstigere Wert zugrundegelegt wurde (Fig. 4).

Beispiel 11: Stabilität von [Ala[1],Thr[2]] Desulfatohirudin-Analogen bei pH 4 und 60°C

Die gereinigten Isohirudine wurden als Lyophilisat zu 1 mg/ml in Wasser gelöst und mit 1M Essigsäure auf pH 4 gestellt. Inkubation und Analysen wurden entsprechend Beispiel 10 durchgeführt (Fig.3).

**Patentansprüche**

1.    Isohirudine mit verbesserter Stabilität gekennzeichnet durch die folgenden Aminosäuren

an Position 33 Glu
an Position 52 Gln, Glu, Asn oder Asp
an Position 53 Glu
an Position 54 Gly oder Ala
an Position 55 Asp oder Glu.

2.    Isohirudine nach Anspruch 1 mit der Formel

A¹      -A² -Tyr-Thr-Asp-Cys-Thr-Glu-Ser-Gly-Gln-Asn-Leu-Cys-Leu-

20                                                           30

Cys-Glu-Gly-Ser-Asn-Val-Cys-Gly-Gln-Gly-Asn-Lys-Cys-Ile-Leu-

40

Gly-Ser- B  - Gly-Glu-Lys-Asn-Gln-Cys-Val-Thr-Gly-Glu-Gly-Thr-

50        52    53    54    55                    60

Pro-Lys-Pro-Gln-Ser-His-  C  -  D  -  E  -  F  -Phe-Glu-Glu-Ile-Pro-

Glu-Glu-Tyr-Leu-Gln

wobei

A¹       = Leu, Ala Ile oder Val bedeutet
A²       = Thr oder Val
B        Glu bedeutet
C        Gln oder Glu bedeutet
D        Glu bedeutet
E        Gly oder Ala bedeutet
und F    Asp oder Glu bedeutet.

3.   Isohirudine nach Anspruch 1 oder 2, wobei Position 1 Ala oder Leu bedeutet, an Position 2 Thr steht, an Position 33 Glu steht, an Position 52 Gln steht, an Position 53 Glu steht, an Position 54 Gly steht und an Position 55 Glu steht.

4.   Isohirudine nach Anspruch 1 oder 2, wobei Position 1 Ala oder Leu bedeutet, an Position 33 Glu steht, an Position 52 Gln steht, an Position 53 Glu steht, an Position 54 Ala steht und an Position 55 Asp steht.

5.   Vektoren, die für Isohirudine gemäß der Ansprüche 1 bis 4 kodieren.

6.   Verfahren zur Herstellung von Isohirudinen nach Anspruch 1 bis 4, dadurch gekennzeichnet, daß Proteine der Wirts-Zelle durch eine saure Fällung bei einem pH ≤ 4 abgetrennt werden, wonach das Produkt aus dem Kulturmedium durch sukzessive chromatographische Reinigung, die zumindest einen Kationenaustauscher und eine RP-Chromatographie umfaßt, hochrein dargestellt wird.

7.   Arzneimittel, die ein oder mehrere Isohirudine gemäß den Ansprüchen 1 bis 4 als Wirkstoff enthalten.

8.   Galenische Zubereitung eines Isohirudins gemäß Ansprüchen 1 bis 4, dadurch gekennzeichnet, daß die Wirksubstanz in einer Konzentration von 1-500 mg/ml und bei einem pH zwischen 4 und 8 in gelöster Form für die parenterale (i.v. und s.c.) Applikation vorliegt.

**Claims**

1.   An isohirudin with improved stability and with the following amino acids

Glu at position 33
Gln, Glu, Asn or Asp at position 52
Glu at position 53
Gly or Ala at position 54
Asp or Glu at position 55.

2.   An isohirudin as claimed in claim 1 with the formula

```
A¹     -A²-Tyr-Thr-Asp-Cys-Thr-Glu-Ser-Gly-Gln-Asn-Leu-Cys-
                    20
Leu-Cys-Glu-Gly-Ser-Asn-Val-Cys-Gly-Gln-Gly-Asn-Lys-
        30                                              40
Cys-Ile-Leu-Gly-Ser- B -Gly-Glu-Lys-Asn-Gln-Cys-Val-Thr-
                            50          52   53   54   55
Gly-Glu-Gly-Thr-Pro-Lys-Pro-Gln-Ser-His- C - D - E - F-
            60
Phe-Glu-Glu-Ile-Pro-Glu-Glu-Tyr-Leu-Gln
```

where

| | |
|---|---|
| A¹ | is Leu, Ala, Ile or Val, |
| A² | is Thr or Val, |
| B | is Glu, |
| C | is Gln or Glu, |
| D | is Glu, |
| E | is Gly or Ala, |
| and F | is Asp or Glu. |

3. An isohirudin as claimed in claim 1 or 2, where position 1 denotes Ala or Leu, Thr is at position 2, Glu is at position 33, Gln is at position 52, Glu is at position 53, Gly is at position 54 and Glu is at position 55.

4. An isohirudin as claimed in claim 1 or 2, where position 1 denotes Ala or Leu, Glu is at position 33, Gln is at position 52, Glu is at position 53, Ala is at position 54 and Asp is at position 55.

5. A vector which codes for an isohirudin as claimed in claims 1 to 4.

6. A process for the preparation of isohirudins as claimed in claim 1 to 4, which comprises separating proteins of the host cell by an acid precipitation at a pH ≤ 4, after which the product is prepared with high purity from the culture medium by successive chromatographic purification which comprises at least one cation exchanger and one RP chromatography.

7. A pharmaceutical which contains one or more isohirudins as claimed in claims 1 to 4 as active ingredient.

8. A pharmaceutical formulation of an isohirudin as claimed in claims 1 to 4, wherein the active substance is present in a concentration of 1-500 mg/ml and at a pH between 4 and 8 in dissolved form for parenteral (i.v. and s.c.) administration.

**Revendications**

1. Isohirudines à stabilité améliorée, caractérisées par les aminoacides suivants

   Glu à la position 33,
   Gln, Glu, Asn ou Asp à la position 52,
   Glu à la position 53,
   Gly ou Ala à la position 54,
   Asp ou Glu à la position 55.

2. Isohirudines selon la revendication 1, de formule

A¹ ‑A² ‑Tyr·Thr·Asp·Cys·Thr·Glu·Ser·Gly·Gln·Asn·Leu·Cys·Leu·

20                                                                    30

Cys·Glu·Gly·Ser·Asn·Val·Cys·Gly·Gln·Gly·Asn·Lys·Cys·Ile·Leu·

40

Gly·Ser· B · Gly·Glu·Lys·Asn·Gln·Cys·Val·Thr·Gly·Glu·Gly·Thr·

50          52   53   54   55                              60

Pro·Lys·Pro·Gln·Ser·His· C · D · E · F ·Phe·Glu·Glu·Ile·Pro·

Glu·Glu·Tyr·Leu·Gln

dans laquelle

A¹    représente Leu, Ala, Ile ou Val,
A²    représente Thr ou Val,
B     représente Glu,
C     représente Gln ou Glu,
D     représente Glu,
E     représente Gly ou Ala et
F     représente Asp ou Glu.

3. Isohirudines selon la revendication 1 ou 2, dans lesquelles la position 1 représente Ala ou Leu, Thr se trouve à la position 2, Glu se trouve à la position 33, Gln se trouve à la position 52, Glu se trouve à la position 53, Gly se trouve à la position 54 et Glu se trouve à la position 55.

4. Isohirudines selon la revendication 1 ou 2, dans lesquelles la position 1 représente Ala ou Leu, Glu se trouve à la position 33, Gln se trouve à la position 52, Glu se trouve à la position 53, Ala se trouve à la position 54 et Asp se trouve à la position 55.

5. Vecteurs qui codent pour les isohirudines selon les revendications 1 à 4.

6. Procédé pour la production d'isohirudines selon les revendications 1 à 4, caractérisé en ce que l'on sépare des protéines de la cellule hôte par une précipitation acide à pH ≤ 4, à la suite de quoi on obtient le produit très pur à partir du milieu de culture par purification chromatographique subséquente, qui comprend au moins un échangeur de cations et une chromatographie en phase inverse

7. Médicament contenant comme substance active une ou plusieurs isohirudines selon les revendications 1 à 4.

8. Composition galénique d'une isohirudine selon les revendications 1 à 4, caractérisée en ce que la substance active est présente sous forme dissoute, pour l'administration parentérale (i.v. et s.c.), à une concentration de 1-500 mg/ml et à un pH entre 4 et 8.

Fig. 1

pCMT 203
6657

EP 0 549 915 B1

VERLÄNGERUNG DER PTZ NACH 0,5 mg I.V. RHESUSAFFE

*Fig. 2*

PTZ [sec]

t [min]

—*— [ Leu$^1$,Thr$^2$ ] DESULFATOHIRUDIN

—+— [ Ala$^1$,Thr$^2$ ] DESULFATOHIRUDIN

—*— [Ala$^1$,Thr$^2$,Glu$^{33}$,Gln$^{52}$,Glu$^{53}$,Glu$^{55}$] DESULFATOHIRUDIN

—⊠— [Ala$^1$,Thr$^2$,Glu$^{33}$,Gln$^{52}$,Glu$^{53}$,Ala$^{54}$] DESULFATOHIRUDIN

**Fig. 3** STRESSINKUBATION, 60°C, pH 4

RELATIVE REINHEIT [%]

t [h]

—*— [Leu¹,Thr²] DESULFATOHIRUDIN

—+— [Ala¹,Thr²] DESULFATOHIRUDIN

----×---- [Ala¹,Thr²,Glu³³,Gln⁵²,Glu⁵³,Glu⁵⁵] DESULFATOHIRUDIN

········⊠········ [Ala¹,Thr²,Glu³³,Glu⁵³] DESULFATOHIRUDIN

—△— [Ala¹,Thr²,Glu³³,Gln⁵²,Glu⁵³,Ala⁵⁴] DESULFATOHIRUDIN

EP 0 549 915 B1

*Fig. 4*  STRESSINKUBATION, 60°C, pH 6,5

RELATIVE REINHEIT [%]

t [h]

————*————  [ Leu¹,Thr²] DESULFATOHIRUDIN

————+————  [ Ala¹,Thr²] DESULFATOHIRUDIN

————×———— [Ala¹,Thr²,Glu³³,Gln⁵²,Glu⁵³,Glu⁵⁵] DESULFATOHIRUDIN

·······⊠······· [Ala¹,Thr²,Glu³³,Glu⁵³] DESULFATOHIRUDIN

————△———— [Ala¹,Thr²,Glu³³,Gln⁵²,Glu⁵³,Ala⁵⁴] DESULFATOHIRUDIN

EP 0 549 915 B1